# EUROPEAN PATENT APPLICATION

(11) **EP 4 659 649 A1**
(43) Date of publication of application: **10.12.2025**
(21) Application number: 25175951.0
(22) Date of filing: 13.05.2025
(51) Int. Cl.: A61B 1/00, A61B 1/31, A61B 1/32

(54) **A RECTOSCOPE FOR INSPECTING A CAVITY**

(30) Priority: 06.06.2024 IT 202400012985
(71) Applicant: THD S.p.A., 42015 Correggio (RE) (IT)
(72) Inventor: BASTIA, Filippo, 41019 Soliera (IT)
(74) Representative: Paparo, Aldo

(57) **Abstract**

A rectoscope (1) for inspecting a cavity of an individual, comprising a dilator body (2), extending prevalently along a longitudinal axis (X), between a proximal end (21) and a distal end (22), wherein the proximal end (21) and the distal end (22) are connected by a side wall (23) and define, together with said side wall (23), an inner volume (V). The dilator body (2) comprises in the proximal end (21) a cover (4) movable between a first position, in which the cover (4) defines a closing of a proximal opening (PO) of the proximal end (21), and a second position in which the cover (4) is moved away from the proximal opening (PO) so as to make the inner volume (V) of the dilator body (2) accessible. The cover (4) comprises at least one window (5) made of a frangible material and breakable in use, when the cover (4) is in said first position, to allow a controlled access to the inner volume (V) of the dilator body (2).

## Description

The present invention relates to a rectoscope for inspecting a cavity.

The present invention is advantageously applied in the field of diagnosis of pathologies relating to human, natural or surgically obtained cavities or orifices, a diagnosis that is performed by means of visual inspection of the tissues delimiting the cavities and the orifices.

The present invention is therefore applicable in the field of anorectal inspection.

In other words, the rectoscope is insertable, in use, into an anorectal cavity of an individual.

Inspection devices comprising a dilator body and an introducer body are known in the prior art: the dilator body is hollow and has a tubular extension between a distal opening to access visually and operatively the mucous membranes delimiting the cavity, and a proximal opening, accessible by the operator at the moment of use; the introducer body also has a tubular extension, between a distal end of substantially ogival shape and a proximal end, graspable by the operator.

The introducer body is configured to be coupled to the dilator body by sliding of the introducer body inside the dilator body, until the ogival end of the introducer body protrudes from the distal opening of the dilator body, obtaining outer walls shaped to facilitate insertion of the inspection device into the cavity of the patient.

For the specific case of rectoscopes, there could be a need to remove the introducer body if the dilator body encounters resistance upon insertion into the device, due to the anal contractions of the patient. In this case, in fact, it is envisaged to insert air into the dilator body so as to dilate the anal walls, thus facilitating dilation of the anal canal and consequent insertion of the dilator body.

In order to avoid the air introduced into the dilator body exiting from the proximal opening and/or bodily fluids or biological materials exiting from the proximal opening during insertion of the dilator body, the use of a cover or another similar device is envisaged, in order to occlude the proximal opening.

In this manner, during the introduction of air into the dilator body, the flow of air will be caused to exit from the distal opening and any fluids will be blocked by the cover itself.

Despite this, there is a risk that, after opening of the cover in order to insert instruments for inspection and/or of another kind, the bodily fluid could exit from the proximal opening due to the different pressures between the inside of the dilator body or the cavity and the outside. In other words, there is a risk that the fluid could exit and hit the surgeon, with consequent discomfort from both a health and hygiene standpoint.

The technical task of the present invention is therefore to provide a rectoscope that is able to overcome the drawbacks that have emerged from the prior art.

An object of the present invention is therefore to provide a rectoscope that prevents undesired exits of fluid also after insertion of the rectoscope into the cavity.

A further object of the present invention is therefore to provide a rectoscope that allows inspection operations and/or other types of operations to be performed without discomfort for the surgeon.

The specified technical task and the specified objects are substantially achieved by a rectoscope comprising the technical features set forth in one or more of the appended claims. The dependent claims correspond to possible embodiments of the invention.

In particular, the specified technical task and the specified objects are substantially achieved by a rectoscope for inspecting a cavity of an individual.

According to an aspect of the present invention, the rectoscope for inspecting a cavity is insertable into an anorectal cavity of the individual.

The rectoscope comprises a dilator body, extending prevalently along a longitudinal axis, between a proximal end and a distal end. The proximal end and the distal end are connected by a side wall and define, together with the side wall, an inner volume.

According to an aspect of the present invention, the proximal end identifies a proximal opening, coaxial with the longitudinal axis and connected to a gripping element, which is accessible or graspable by an operator.

According to an aspect of the present invention, the distal end identifies a distal opening, for access to the tissues delimiting the cavity from the inner volume.

The dilator body comprises in the proximal end a cover movable between a first position and a second position.

In the first position, the cover defines a closing (preferably a hermetic seal) of the proximal opening. In other words, the first position of the cover allows introduction without returns of fluid through the proximal opening of the dilator body.

In the second position, the cover is moved away from the proximal opening so as to make the inner volume of the dilator body accessible. In other words, in the second position of the cover, it is possible to access the inner volume in order to be able to introduce instruments for inspection and/or medical instruments of various kinds.

According to an aspect of the invention, the dilator body further comprises, in the proximal end, a cannula adapted to favour, when the cover is in the first position, a communication of air from the outside of the dilator body to the inner volume. According to an aspect of the present invention, the rectoscope comprises a pump or another similar instrument connected or connectible to the cannula and configured to produce a flow of air that, when the cover is in the first position, allows a thrust action to be generated through the distal opening that allows dilation of the cavity so as to facilitate an insertion of the dilator body.

According to an aspect of the present invention, the rectoscope further comprises an introducer body, having a gripping portion.

The gripping portion, accessible or graspable by the operator, is connected to an insertion portion having an elongated extension along the longitudinal axis between an insertion end, of substantially ogival shape, distal with respect to the gripping portion, and an intermediate portion proximal with respect to the gripping portion.

The insertion portion is slidably insertable within, and disengageable from, the inner volume through the proximal opening when the cover is in the second position.

According to an aspect of the present invention, the cover comprises at least one window made of a frangible material and breakable in use, when the cover is in the first position, to allow a controlled access to the inner volume of the dilator body.

Preferably, the cover comprises at least two windows, arranged in opposite portions of the cover with respect to a central portion of the cover itself.

In other words, the windows (or frangible windows) allow the insertion of medical instruments of reduced dimensions that, by breaking the windows themselves during their insertion, avoids undesired returns of fluid from occurring.

Preferably, the at least one window is obtained as a hole sealed by means of a film.

Preferably, the at least one window is delimited by or obtained by means of weakening lines defined on the cover itself.

According to an aspect of the present invention, the dilator body is made of a transparent material.

According to an aspect of the present invention, the cover is made of a transparent material. Therefore, the at least one window (or the windows) is made of a transparent material.

Further characteristics and advantages of the present invention will become more apparent from the indicative and thus non-limiting description of an embodiment of a rectoscope.

Such a description will be set out below with reference to the accompanying drawings, which are provided solely for illustrative and therefore non-limiting purposes, in which:
- Figure 1 is a perspective schematic view of the rectoscope subject-matter of the present invention in a first configuration of use;
- Figure 2 is a perspective schematic view of the rectoscope subject-matter of the present invention in a second configuration of use;
- Figure 3 is a sectional schematic view of the rectoscope subject-matter of the present invention in a third configuration of use;
- Figure 4 is a sectional schematic view of the rectoscope subject-matter of the present invention according to a further embodiment;

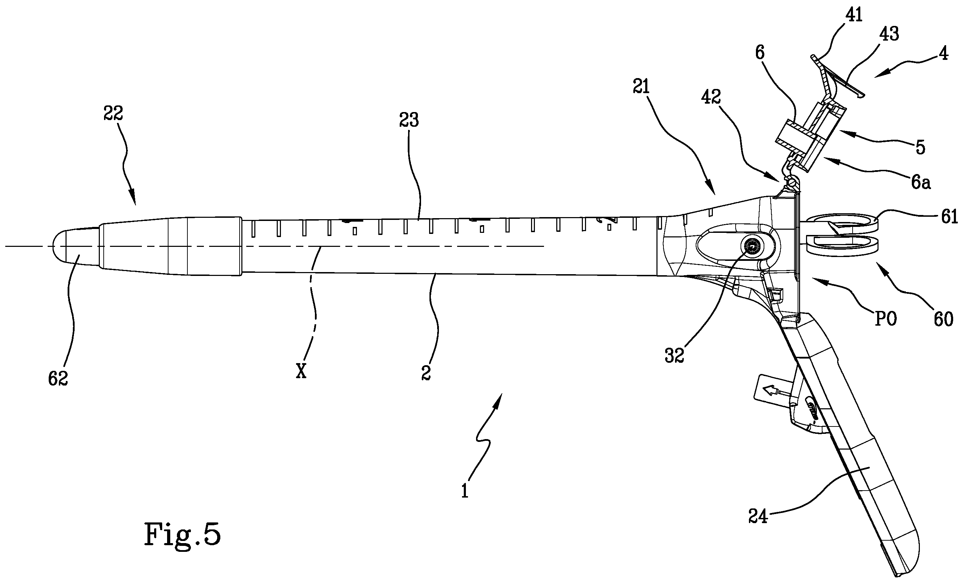

- Figure 5 is a schematic side view of the rectoscope subject-matter of the present invention according to a further embodiment;
- Figure 6 is a perspective schematic view of the rectoscope of the present invention according to a further embodiment.

With reference to the appended figures, the number 1 indicates overall a rectoscope.

The rectoscope 1 is insertable, in use, into a cavity of an individual. In particular, the rectoscope 1 is insertable into an anorectal cavity of an individual.

The rectoscope 1 is a device for inspecting the aforesaid cavity by an operator. The rectoscope 1 is therefore usable for inspecting a rectal or anorectal cavity of a patient.

The terms "distal" and "proximal" will be used in the description. Such terms are, in this context, to be intended as with respect to the operator: the term "proximal" is to be intended as referring to the parts furthest away from the patient (or those closest to the operator), whereas the term "distal" is to be intended as referring to the parts closest to the patient (or those furthest from the operator).

The rectoscope 1 comprises a dilator body 2 extending prevalently along a longitudinal axis "X". In particular, the dilator body 2 extends between a proximal end 21 and a distal end 22. In other words, the dilator body 2 has an elongated extension along the longitudinal axis "X" defining two ends, the proximal one 21 and the distal one 22.

The proximal end 21 and the distal end 22 are connected by a side wall 23 and define, together with the side wall 23, an inner volume "V". In other words, the side wall 23 delimits and identifies, together with the proximal end 21 and the distal end 22, the inner volume "V".

Preferably, the rectoscope 1 (or at least the dilator body 2) is made of transparent polymeric material, in order to allow the view of the tissues and surrounding mucous membranes.

Preferably, the dilator body 2 extends with a substantially tubular shape. Preferably, the dilator body 2 extends in a diverging fashion from the proximal end 21 to the distal end 22. In other words, the dilator body 2 extends in a converging fashion from the distal end 22 to the proximal end 21. Advantageously, such shape of the dilator body 2 makes insertion of the dilator body 2 itself into the cavity to be explored easier.

The proximal end 21 identifies a proximal opening "PO", coaxial with the longitudinal axis "X".

Similarly, the distal end 22 identifies a distal opening "DO", preferably coaxial with the longitudinal axis "X", configured to allow access, from the inner volume "V", to the tissues and to the mucous membranes delimiting the cavity.

The proximal opening "PO" and the distal opening "DO" have a substantially circular shape.

The proximal end 21 is connected to a gripping element 24, accessible or graspable by an operator. In other words, the gripping element 24 extends transversely with respect to the longitudinal axis "X".

Preferably, the rectoscope 1 comprises lighting means 3 to generate a direct light beam from the proximal end 21 towards the distal end 22.

The lighting means 3 is preferably defined by a LED light.

Preferably, the dilator body 2 has a cavity 25 obtained inside it and at the proximal end 21.

The lighting means 3 is entirely contained in the cavity 25 to generate the light beam directly inside the dilator body 2. In other words, the lighting means 3 is capable of generating a light beam that, thanks to the material of which the dilator body 2 itself is made (generally a transparent material or one with reduced opacity), is capable of lighting the inner volume "V" or the distal opening "DO".

In other words, in proximity to the proximal opening "PO" or in proximity to the proximal end 21, a cavity 25 is defined within which the lighting means 3 is inserted. The cavity 25 has an at least partially elongated shape and extends along an axis incident, at least in proximity to the distal opening "DO", with the longitudinal axis "X". In this manner, the lighting means 3 is oriented so as to light the inner volume "V" or the distal opening "DO". Therefore, in this embodiment of the rectoscope 1, the lighting means 3 positioned in the cavity 25 defines an indirect light source of the distal opening "DO" or the inner volume "V".

Preferably, the gripping element 24 comprises a first element 24a, solidly constrained to the dilator body 2, and a second element 24b couplable to the first element 24a.

Preferably, the gripping element 24 is made in the form of a handle. Even more preferably, the handle is defined by the first element 24a and by the second element 24b.

Preferably, the second element 24b is, in turn, defined by a first half-shell "S1" and by a second half-shell "S2".

With particular reference to the first element 24a, this comprises a seat 50, extending for the entire extension of the first element 24a, to house the second element 24b.

The seat 50 is interrupted by a window 51 adapted to receive a corresponding protrusion, solidly constrained to the second element 24b, to favour the connection and coupling stability between the first and second element 24a, 24b.

Preferably, the second element 24b has a termination 24c adapted to accommodate and to retain the lighting means 3.

In other words, the lighting means 3 is positioned inside the cavity 25 by means of the second element 24b.

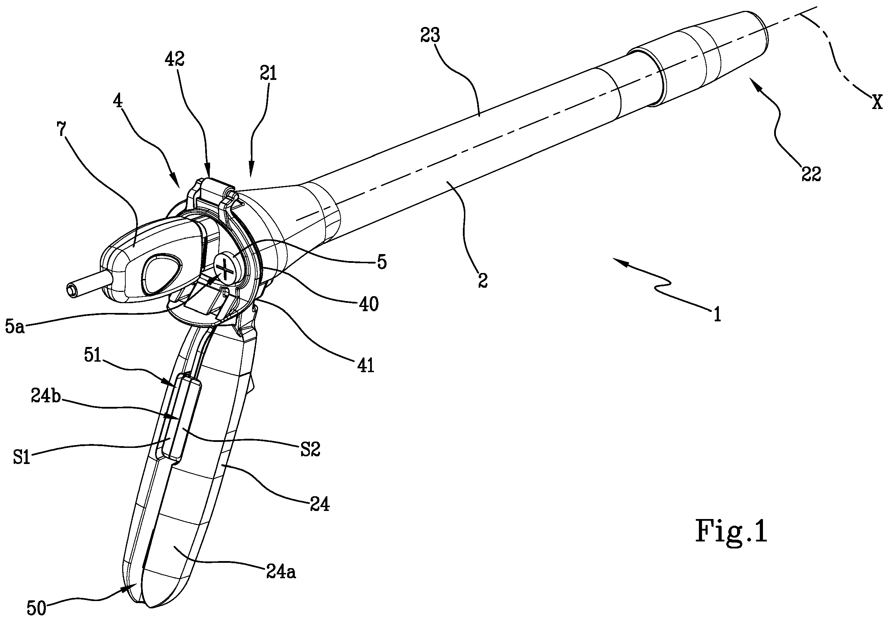

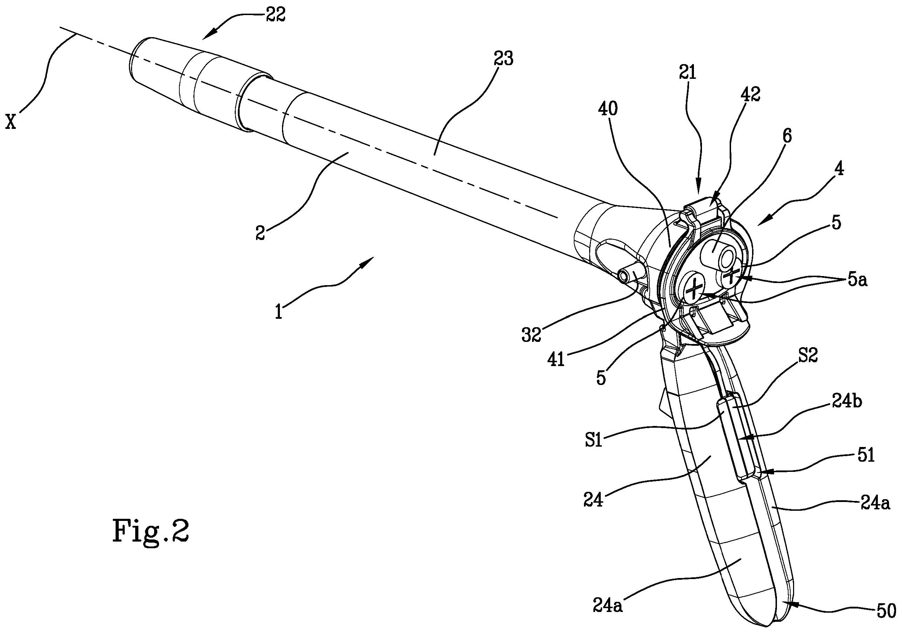

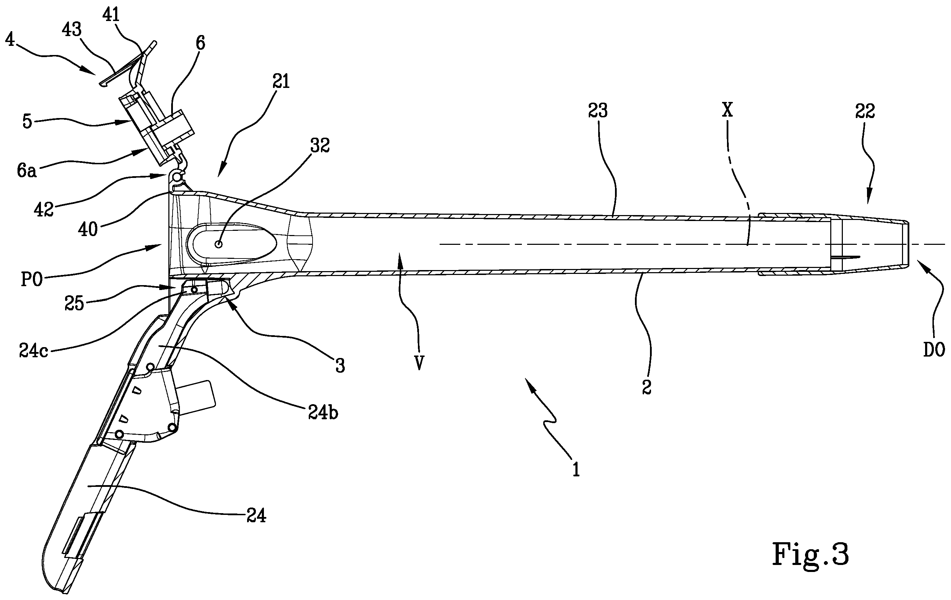

The dilator body 2 comprises in the proximal end 21 a cover 4 movable between a first position (Figures 1 and 2) and a second position(Figure 3). In the first position, the cover 4 defines a closing (preferably a hermetic seal) of the proximal opening "PO". In other words, in the first position the cover 4 prevents access to the inner volume "V". Therefore, in the first position the cover 4 is transverse or perpendicular to the longitudinal axis "X". In the first position, the cover 4 is preferably locked by means of interlocking at the proximal end 21.

In the second position, the cover 4 is moved away from the proximal opening "PO" so as to make the inner volume "V" of the dilator body 2 accessible.

Preferably, the cover 4 can be removed entirely from the proximal end 21. Preferably, the cover 4 can be at least partially separated from the proximal end 21.

Preferably, the cover 4 comprises a base 40 coupled or defined at the proximal end 21 and an overturning portion 41 movable between the first and the second position with respect to the base 40 by means of a hinge coupling 42. In other words, the hinge coupling 42 defines a rotation axis for the overturning portion 41 with respect to the base 40.

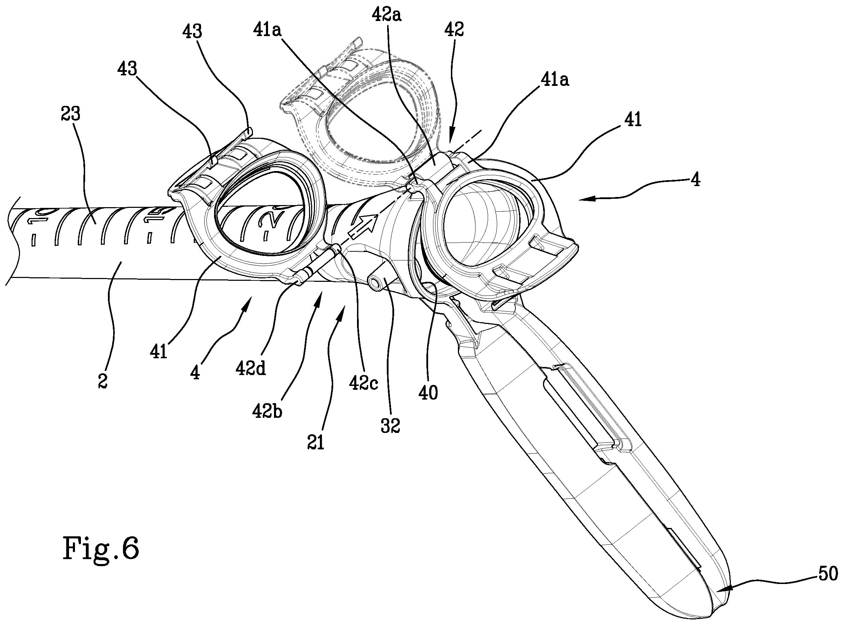

In one embodiment, for example the one of Figure 6, the hinge coupling 42 has a guide 42a having a C-shaped section and a rotation pin 42b insertable, in use, into the guide 42a. In particular, the rotation pin 42b extends between two ends, wherein a first end 42c defines a portion for insertion of the rotation pin 42b into the guide 42a and a second end 42d defines a limit stop portion of the rotation pin 42b during its insertion into the guide 42a.

As shown in the appended figures, the first end 42c has a diameter that is lower than the diameter of the second end 42d so as to allow insertion of the rotation pin 42b into the guide 42a.

The rotation pin 42b also has connecting arms 41a between the rotation pin 42b itself and the overturning portion 41. In particular, the two arms 41a are associated with the two ends of the rotation pin 42b. During an insertion of the rotation pin 42b into the guide 42a, the connecting arms 41a do not interfere with insertion into the guide 42a and, on the contrary, at least the one associated with the second end 42d is also capable of sliding inside the guide 42a.

Once the overturning portion 41 has been overturned, the connecting arms 41a will prevent an undesired removal of the cover 4 by interfering with the sliding of the rotation pin 42b.

Preferably, the overturning portion 41 can be provided with an interlocking tooth 43 that, in the first position, can be interlocked in the cavity 25 or in another portion of the proximal end 21 suited to the task.

The dilator body 2 preferably comprises, in the proximal end 21, a cannula 32 adapted to favour, when the cover 4 is in the first position, a communication of air from the outside of the dilator body 2 to the inner volume "V".

Preferably, the rectoscope 1 comprises a pump or other similar instrument (not shown) connected or connectible to the cannula 32 and configured to produce a flow of air that, when the cover 4 is in the first position, allows a thrust action to be produced through the distal opening "DO" that allows dilation of the cavity so as to facilitate an insertion of the dilator body 2. In other words, the rectoscope 1 comprises a pumping device communicating, in use, with the cannula 32 and configured to produce a flow of air inside the inner volume "V" apt to define the thrust action through the distal opening "DO".

The cover 4 or the at least one window 5 prevents, when the cover 4 is in the first position, an exit of air from the inner volume "V" to the outside of the dilator body 2 through the proximal opening "PO".

Preferably, the rectoscope 1 further comprises an introducer body 60, having a gripping portion 61, accessible or graspable by the operator, connected to an insertion portion having an elongated extension along the longitudinal axis "X" between an insertion end 62, of substantially ogival shape, distal with respect to the gripping portion 61, and an intermediate portion proximal with respect to the gripping portion 61.

The insertion portion is slidably insertable within, and disengageable from, the inner volume "V" through the proximal opening "PO" when the cover 4 is in the second position.

In use, the introducer body 60 is used in a first step of inserting the rectoscope 1 into the anal cavity and, subsequently, is removed and the cover 4 is made to close so as to occlude the proximal opening "PO", in order to be able to pump air safely through the cannula 32 and thus produce the aforesaid thrust action through the distal opening "D", which allows dilation of the anorectal cavity in order to allow correct insertion, for its entire length, of the rectoscope 1, or the dilator body 2.

With reference to the appended figures, the cover 4 (or the overturning portion 41) of the rectoscope 1 comprises at least one window 5 (or frangible window 5) made of frangible material. The window 5 is breakable in use, when the cover 4 is in the first position, to allow a controlled access to the inner volume "V" of the dilator body 2.

In particular, it is possible to perforate the window 5 with appropriate tools, in order to allow the cavity to be inspected. Advantageously, this solution avoids the return of undesired fluids occurring. The difference in pressure that would cause returns of bodily fluids and/or organic materials that would occur with direct opening of the cover 4 would, in fact, be reduced, and any fluids/materials would not exit or their exit would, in any case, be highly limited.

Fracturing of the window 5 does, in fact, allow depressurising of the inner volume "V" in a manner that the fluids cannot exit from the window itself, as will be clearer from the continuation of the present description.

Furthermore, the window 5 is easily frangible by means of inspection instruments, so as to prevent discomfort for the individual caused by further undesired thrusts. In other words, the window 5 is made of a material resistant to the pressure exerted while producing the flow of air, but easily breakable by means of the inspection instruments, so as to prevent undesired thrusts by the rectoscope 1 itself inside the anorectal cavity of the individual.

Preferably, the cover 4 comprises at least one pair of windows 5 opposite to each other with respect to a central portion of the cover 4 itself. In this manner, simultaneous insertion of two inspection instruments inside the inner volume "V" is possible.

Preferably, the cover 4 (or the overturning portion 41) comprises a plurality of windows 5 or pairs of windows 5, as a function of an extension of the cover 4 itself or the type of inspections that need to be performed with specific instruments.

Preferably, the window 5 (or the windows 5) has a circular shape. Preferably, the window 5 (or the windows 5) has a quadrangular shape. Preferably, the window 5 (or the windows 5) can have any shape that, in use, does not impede access of the inspection instruments.

Preferably, the at least one window 5 is made in the shape of a hole obtained on the cover 4.

The hole, obtained on the cover 4, is preferably aligned with the longitudinal axis "X".

Preferably, the hole extends along its own axis, incident with respect to the longitudinal axis "X" and directed towards the distal opening "DO" so as to facilitate an exit of the inspection instruments towards the distal opening "DO" itself. In the embodiment in which two windows 5 are present, both holes are oriented towards the distal opening "DO".

In said embodiment, the hole is occluded by a film. Preferably, the film is obtained in low-density polyethylene. Other materials with similar characteristics are usable for making the film. Preferably, the film is made of a transparent material, so that a user of the inspection instruments can check that it is safe to perforate the film.

Preferably, the at least one window is obtained or delimited by weakening lines 5a such as those shown, for example, in the appended figures.

The weakening lines 5a can be obtained on the cover 4 itself.

The weakening lines 5a can alternatively be obtained on a cap or other covering element positioned to occlude a hole or specific seat defined on the cover 4. Preferably, the cap or other covering element can be made of PMMA (polymethylmethacrylate).

Preferably, the weakening lines 5a define or are made in the shape of a cross delimiting the window 5 itself.

Preferably, the pair of windows 5 can both be made in the shape of a hole occluded by a film.

Preferably, the pair of windows 5 can both be delimited or obtained by means of weakening lines 5a.

Preferably, one window 5 of the pair of windows 5 can be made in the shape of a hole occluded by a film and the other window 5 of the pair can be delimited or made by means of weakening lines 5a.

Preferably, considering a circular extension of the cover 4 (or of the overturning portion 41), the windows 5 are defined laterally to a central portion of the cover 4 itself. Therefore, the windows 5 are arranged between two semi-circles defining the cover 4 itself.

The windows 5 are preferably defined in "central and lateral" portions of the cover 4 if it were to have different shapes.

In this manner, any bodily fluids and/or organic materials that should be deposited towards the proximal opening "PO" would be contained below the windows 5 and therefore, once they have been perforated, would not be caused to exit from the proximal opening "PO" through the windows 5 themselves.

Preferably, the cover 4 (or the overturning portion 41) is provided with a connection seat 6. Preferably, the connection seat 6 is defined in an upper central portion of the cover 4 (or of the overturning portion 41).

Preferably, the windows 5 are defined/made in lateral portions with respect to the connection seat 6.

The rectoscope 1 preferably comprises an acquisition device 7 configured at least to acquire images in a portion external to the distal opening "DO". In other words, the rectoscope 1 defines with the acquisition device 7 a kit for inspecting the cavity of the individual.

The acquisition device 7 is therefore connectible, in use, to the connection seat 6. In other words, the acquisition device 7 or a portion thereof used for acquisition of images is shaped so as to fit onto or be inserted into the connection seat 6.

Preferably, the connection seat 6 is provided with a transparent window or a lens (indicated overall with number 6a in figure 3) defining an acquisition axis passing through the distal opening "DO". Preferably, the acquisition axis is transverse to the longitudinal axis "X".

Preferably, the connection seat 6, or the transparent window or lens, also allows observation of the anorectal canal with the naked eye, so that the acquisition device 7 does not necessarily have to be used.

The positioning of the connection seat 6 allows, during an insertion of the inspection instruments through the window 5 (or the windows 5) the acquisition of images (or videos) by means of the acquisition device 7 not to be occluded by them.

In a further embodiment of the rectoscope 1, it further comprises a lighting device 8 configured to generate a light beam passing through the proximal opening "PO" and the distal opening "DO".

Preferably, the lighting device 8 is integral with the acquisition device 7, defining an inspection unit 100.

The at least one connection seat 6 is also configured, in use, to allow connection of the lighting device 8 to the central portion of the cover 4. In other words, the connection seat 6 can be shaped so as to allow the simultaneous connection of both the acquisition device 7 and the lighting device 8.

Preferably, where the lighting device 8 and the acquisition device 7 are integral with each other, the connection seat 6 allows connection of the inspection unit 100.

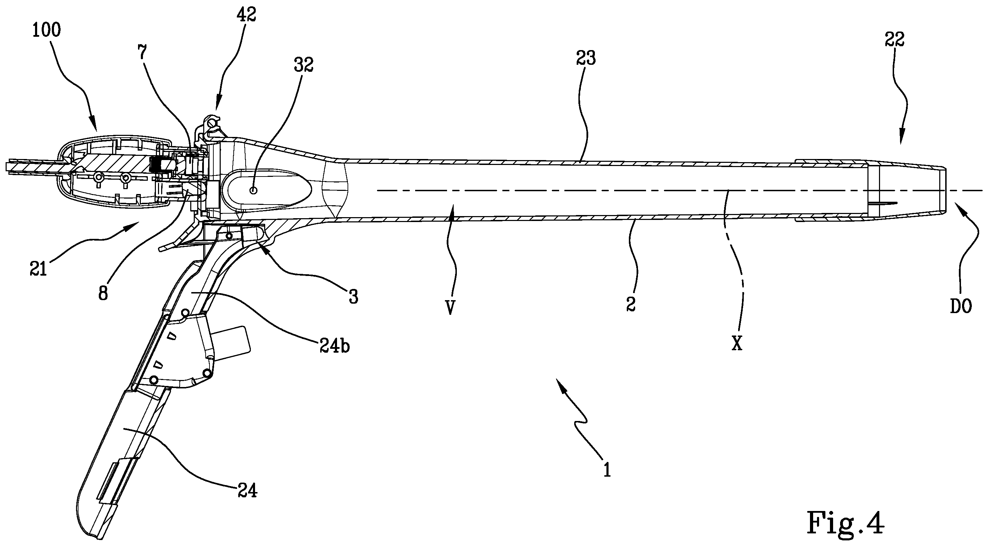

Alternatively, the connection seat 6 is configured to maintain the lighting device 8 in proximity to the central portion of the cover 4. For example, with particular reference to the embodiment in which an inspection unit 100 is defined, the portion of inspection unit 100 in which the acquisition device 7 is defined is shaped so as to fit onto or be inserted into the connection seat 6 and the lighting device 8 (arranged, in use, below the acquisition device 7, as shown in Figure 4) is consequently positioned facing the cover 4 towards the distal opening "DO". In other words, the connection seat 6 is arranged on the cover 4 so as to define a space (preferably transparent) suited to the passage of the light beam generated by the lighting device 8.

The lighting device 8 therefore defines a direct light source for the distal opening "DO" or the inner volume "V".

According to one embodiment of the rectoscope 1, it comprises solely the lighting means 3.

According to a further embodiment of the rectoscope 1, it comprises solely the lighting device 8 (both whether or not it is integral with the acquisition device 7).

According to a further embodiment of the rectoscope 1, it comprises both the lighting means 3 and the lighting device 8. In this manner, it is possible to best light the inner volume "V" so as to facilitate the inspection operations.

Furthermore, the positioning of the connection seat 6 makes it possible to prevent it (or the transparent window or lens) from entering into contact (or excessively into contact) with any fluids and/or organic materials that would occlude its acquisition axis.

Preferably, in a further embodiment, the cover 4 (or the overturning portion 41) does not have the seat and may be provided with a transparent window or a defining lens with which an operator can observe inside the volume "V" or observe beyond the distal opening "DO" if it is necessary to inspect with the naked eye.

Advantageously, the rectoscope 1 subject-matter of the present invention is able to overcome the drawbacks which have emerged from the prior art. Advantageously, the present invention prevents undesired exits of fluid even after insertion of the rectoscope 1 into the cavity.

Advantageously, the rectoscope 1 allows inspection operations and/or other types of operations to be performed without discomfort for the surgeon.

In other words, the rectoscope 1 subject-matter of the present invention makes it unnecessary to have to open the cover 4 to perform the inspection operations, thus preventing the aforesaid undesired exits of fluids and/or organic materials.

## Claims

1. A rectoscope (1) for inspecting a cavity of an individual, comprising:
a dilator body (2), extending prevalently along a longitudinal axis (X), between a proximal end (21) and a distal end (22), wherein the proximal end (21) and the distal end (22) are connected by a side wall (23) and
define, together with said side wall (23), an inner volume (V);
wherein the proximal end (21) identifies a proximal opening (PO) coaxial with said longitudinal axis (X) and is connected to a gripping element (24), which is accessible or graspable by an operator;
wherein the distal end (22) identifies a distal opening (DO), for access to the tissues delimiting said cavity from the inner volume (V);
said dilator body (2) comprising in said proximal end (21) a cover (4) movable between a first position, in which said cover defines a closing of said proximal opening (PO), and a second position in which said cover (4) is moved away from the proximal opening (PO) so as to make said inner volume (V) of the dilator body (2) accessible;
**characterised in that** said cover (4) comprises at least one window (5) made of a frangible material and breakable in use, when said cover (4) is in said first position, to allow a controlled access to the inner volume (V) of the dilator body (2).

2. The rectoscope (1) according to claim 1, wherein said dilator body (2) also comprises, in said proximal end (21), a cannula (32) adapted to favour, when said cover is in said first position, a communication of air from the outside of the dilator body (2) to the inner volume (V), said cover (4), or said at least one window (5) preventing, when said cover (4) is in said first position, an exit of air from the inner volume (V) towards the outside of the dilator body (2) through said proximal opening (PO).

3. The rectoscope (1) according to claim 1 or 2, wherein said cover (4) comprises at least one pair of windows (5) opposite with respect to a central portion of the cover (4) itself.

4. The rectoscope (1) according to one or more of the preceding claims, wherein said at least one window (5) is made in the form of a hole made on said cover (4), said hole being occluded by a film.

5. The rectoscope (1) according to one or more of claims 1 to 3, wherein said at least one window (5) is made or delimited by weakening lines (5a), said weakening lines (5a) preferably defining a cross delimiting the window (5) itself.

6. The rectoscope (1) according to one or more of the preceding claims, further comprising an acquisition device (7) configured to acquire images in a portion external to said distal opening (DO) and wherein said cover (4) further comprises at least one connection seat (6) for said acquisition device (7), defined in a central portion of the cover (4) itself, and provided with a transparent window or a lens (6a) defining an acquisition axis passing through the distal opening (DO).

7. The rectoscope (1) according to claim 6, further comprising a lighting device (8) configured to generate a light beam passing through the proximal opening (PO) and the distal opening (DO) and wherein said at least one connection seat (6) is also configured, in use, to allow the connection or the maintaining of said lighting device (8) in said central portion of the cover (4).

8. The rectoscope (1) according to one or more of the preceding claims, wherein said cover (4) comprises a base (40) coupled or defined at the proximal end (21) and an overturning portion (41) movable between the first and the second position with respect to the base (40) by means of a hinge coupling (42).

9. The rectoscope (1) according to one or more of the preceding claims, comprising lighting means (3) to generate a direct light beam from the proximal end (21) towards the distal end (22) and wherein said dilator body (2) has a cavity (25) obtained inside it, at the proximal end (21), said lighting means (3) being contained inside said cavity (25) to generate said light beam directly inside the dilator body (2) or the inner volume (V).

10. The rectoscope (1) according to one or more of the preceding claims, wherein said gripping element (24) comprises a first element (24a), solidly constrained to the dilator body (2), and a second element (24b) couplable to the first element (24a).

11. The rectoscope (1) according to claim 9 and 10, wherein said second element (24b) has a termination (24c) adapted to accommodate and to retain said lighting means (3).

12. The rectoscope (1) according to one or more of the preceding claims, further comprising an introducer body (60), having a gripping portion (61), accessible or graspable by the operator, connected to an insertion portion having an elongated extension along the longitudinal axis (X) between an insertion end (62), of substantially ogival shape, distal with respect to the gripping portion (61), and an intermediate portion proximal with respect to the gripping portion (61), said insertion portion being slidably insertable within, and disengageable from, the inner volume (V) through the proximal opening (PO) when said cover (4) is in the second position.
